# FASCICULE DE BREVET EUROPEEN

(11) **EP 2 673 028 B1**
(45) Date de publication et mention de la délivrance du brevet: **23.03.2016**
(21) Numéro de dépôt: 12708890.4
(22) Date de dépôt: 09.02.2012
(51) Int. Cl.: A61M 5/178, G21F 5/018, G21F 5/08, A61N 5/10

(54) **DISPOSITIF DE RADIOPROTECTION POUR SERINGUE**
STRAHLENSCHUTZVORRICHTUNG FÜR EINE SPRITZE
RADIATION PROTECTION DEVICE FOR A SYRINGE

(30) Priorité: 10.02.2011 FR 1151088
(43) Date de publication de la demande: 18.12.2013
(73) Titulaire: LEMER PROTECTION ANTI-X PAR ABREVIATION SOCIETE LEMER PAX, 44470 Carquefou (FR)
(72) Inventeur: LEMER, Pierre-Marie, F-44000 Nantes (FR)
(74) Mandataire: Coralis Harle
(86) Numéro de dépôt international: PCT/FR2012/050284
(87) Numéro de publication internationale: WO 2012/168586

(56) Documents cités:
- WO-A1-2004/036597
- US-A- 4 060 073
- US-A- 4 401 108

## Description

La présente invention concerne le domaine général de la radioprotection. Elle concerne plus précisément les dispositifs de radioprotection du type « protège-seringue » qui sont destinés à équiper les seringues servant à l'injection de produit(s) radioactif(s) pour la protection des opérateurs envers les rayonnements ionisants.

Certains secteurs d'activité requièrent la manipulation de produits radioactifs, émetteurs de rayonnements ionisants, tels que les rayonnements de nature électromagnétique (X, gamma) et/ou de nature particulaire (alpha, bêta, neutrons).

Dans le domaine particulier de la médecine nucléaire, des produits radioactifs sont employés pour la mise en oeuvre de techniques de diagnostic et/ou de traitement, telles que l'imagerie fonctionnelle in vivo (par exemple la scintigraphie), le diagnostic biologique in vitro (notamment la radio-immunologie) et la radiothérapie métabolique.

L'administration de ces produits radioactifs au patient est souvent réalisée au moyen d'une seringue sur laquelle est rapporté un dispositif de radioprotection apte à atténuer les rayonnements ionisants notamment pour protéger l'opérateur manipulant cette seringue.

Un tel dispositif de radioprotection, couramment dénommé « protège-seringue », est par exemple décrit dans les documents EP-1 317 299 ou encore US-4 060 073.

Ce genre de dispositif comprend une enveloppe tubulaire de radioprotection qui comporte - une surface intérieure, destinée à recouvrir au moins une partie de la surface extérieure du corps cylindrique de la seringue, et - une surface extérieure, destinée à être prise en main par l'opérateur.

Cette enveloppe tubulaire comprend - une ouverture avant, au travers de laquelle est destinée à déboucher l'extrémité avant du corps cylindrique de la seringue munie d'un orifice d'aspiration et d'éjection d'un liquide, - une ouverture arrière, pour l'introduction et l'extraction dudit corps cylindrique de la seringue, ainsi que pour la manoeuvre de son piston, et, bien souvent - un écran transparent en matériau radio-protecteur, pour accéder visuellement à la surface extérieure du corps cylindrique de la seringue rapportée (pour visualiser le niveau de l'extrémité avant du piston au sein du corps de seringue).

Lorsque cette enveloppe tubulaire est entièrement réalisée dans un matériau radio-protecteur métallique, par exemple en plomb ou en tungstène, cette structure métallique génère des inconvénients sur différents aspects.

Tout d'abord, ces enveloppes tubulaires métalliques ne sont pas complètement efficaces pour protéger la seringue rapportée contre les chocs, notamment en cas de chute. De telles enveloppes métalliques n'apportent donc pas une solution optimale pour la prévention des risques de cassures de la seringue.

D'autre part, l'aspect de ces enveloppes tubulaires métalliques n'est généralement pas de nature à rassurer le patient (effet de stress) et ces enveloppes sont souvent peu agréables au toucher.

De plus, elles sont difficilement personnalisables et peu esthétiques, sur le plan de la couleur notamment. De ce fait, il est parfois fastidieux pour les opérateurs d'identifier le protège-seringue qui est adapté à la seringue à protéger.

Un autre inconvénient réside dans le fait que le corps de la seringue glisse facilement dans la surface intérieure de l'enveloppe radioprotectrice, et qu'il peut être nécessaire de prévoir des moyens complémentaires pour la solidarisation amovible de cette enveloppe avec la seringue.

De son côté, le document US-4 060 073 prévoit une enveloppe radioprotectrice formée d'une coque interne en matière plastique rigide, recouverte par un corps cylindrique en matériau radioprotecteur, lui-même recouvert par une coque externe en matière plastique rigide.
Ici, la coque externe en matière plastique rigide ne protège pas efficacement la seringue contre les chocs. En outre, la coque plastique interne n'est pas adaptée pour empêcher efficacement la seringue de glisser.

Un autre dispositif de radioprotection destiné à équiper une seringue servant à l'injection de produits radioactifs est donné dans US 4 401 108A.

Pour pallier à ces inconvénients, la demanderesse a développé une nouvelle structure de dispositif protège-seringue qui assure une protection efficace de la seringue associée envers les chocs, qui apporte une qualité perçue intéressante, une qualité de toucher améliorée et une possibilité de personnalisation élargie, et qui assure de manière très simple une action efficace de maintien de la seringue.

Le dispositif radio-protecteur correspondant est du type comprenant une enveloppe tubulaire de radioprotection qui comporte - une surface intérieure, destinée à recouvrir au moins une partie de la surface extérieure du corps cylindrique de la seringue, - une surface extérieure, - une ouverture avant, au travers de laquelle est destinée à déboucher l'extrémité avant dudit corps cylindrique, et - une ouverture arrière, pour l'introduction et l'extraction dudit corps cylindrique, ainsi que pour la manoeuvre du piston.
Et cette enveloppe tubulaire comporte encore une pièce extérieure tubulaire, constituant une partie au moins de sa surface extérieure (avantageusement l'intégralité ou la quasi-intégralité de cette surface extérieure d'enveloppe), qui est réalisée en matériau plastique. Conformément à l'invention, ladite pièce extérieure tubulaire est réalisée en matériau élastomère, dont la valeur de dureté Shore A est avantageusement comprise entre 30 et 80 ; de plus, cette pièce extérieure comporte une partie annulaire constrictive comprenant une surface intérieure qui est déformable élastiquement, laquelle surface intérieure constitue un tronçon de la surface intérieure de l'enveloppe tubulaire et est destinée à venir exercer une force de serrage autour du corps cylindrique d'une seringue rapportée, ladite partie annulaire constrictive étant apte à subir une déformation élastique entre deux configurations :
- une configuration active, dans laquelle sa surface intérieure est apte à venir exercer une force de serrage autour du corps cylindrique d'une seringue rapportée, pour générer une force de frottement s'opposant à la translation de l'enveloppe par rapport audit corps cylindrique, et
- une configuration inactive, obtenue par déformation (en particulier manuelle), dans laquelle sa surface intérieure génère une force de serrage réduite autour dudit corps cylindrique, pour limiter, voire supprimer, la force de frottement s'opposant à la translation de l'enveloppe par rapport au corps cylindrique de seringue.

De préférence, cette partie annulaire constrictive constitue l'ouverture arrière de l'enveloppe tubulaire.

Selon une autre particularité, cette partie annulaire constrictive est avantageusement apte à subir une déformation élastique entre deux configurations :
- une configuration active, dans laquelle sa surface intérieure est apte à venir exercer une force de serrage autour du corps cylindrique d'une seringue rapportée, pour générer une force de frottement s'opposant à la translation de l'enveloppe par rapport audit corps cylindrique, et
- une configuration inactive, obtenue par déformation (en particulier manuelle), dans laquelle sa surface intérieure génère une force de serrage réduite autour dudit corps cylindrique, pour limiter, voire supprimer, la force de frottement s'opposant à la translation de l'enveloppe par rapport au corps cylindrique de seringue.

La surface intérieure de l'enveloppe comporte de préférence un tronçon présentant une section circulaire définissant un diamètre donné, correspondant avantageusement, au jeu près, au diamètre du corps cylindrique de la seringue rapportée et prolongeant la partie annulaire constrictive ; et au repos, la surface intérieure de cette partie annulaire constrictive définit un orifice de forme générale allongée, comportant, d'une part, une petite dimension qui est inférieure au diamètre dudit tronçon circulaire de l'enveloppe et, d'autre part, une grande dimension qui est supérieure au diamètre de ce tronçon circulaire d'enveloppe.

Selon cette forme de réalisation, la partie annulaire constrictive comporte avantageusement, au niveau de sa surface extérieure, deux zones d'appui qui sont agencées de manière diamétralement opposée et sur un plan passant par la grande dimension de l'orifice de ladite partie annulaire constrictive, pour faciliter la déformation de cette dernière dans sa configuration inactive.
Les deux zones d'appui en question comportent avantageusement une partie concave, pour réduire localement l'épaisseur de la partie annulaire constrictive et ainsi également réduire la force nécessaire à sa déformation.

De préférence, l'enveloppe tubulaire comprend un écran en matériau radio-protecteur transparent, pour accéder visuellement à la surface extérieure du corps cylindrique de la seringue.

Selon encore une autre particularité, l'enveloppe tubulaire comporte une pièce intérieure tubulaire réalisée en matériau radio-protecteur, logée dans la pièce extérieure et destinée à constituer une partie au moins de la surface intérieure de ladite enveloppe.
Cette pièce intérieure tubulaire comporte avantageusement, sur une partie au moins de sa longueur, une ouverture longitudinale en regard de laquelle est positionné l'écran transparent précité en matériau radio-protecteur.
L'ouverture latérale correspondante est de préférence délimitée par deux bordures longitudinales qui sont chacune munies d'une feuillure pour l'emboîtement de la surface intérieure de l'écran transparent.

La pièce extérieure comporte avantageusement un logement pour l'écran transparent, et le cas échéant un logement pour la pièce intérieure pour leur assemblage sans moyens complémentaires de solidarisation.

Dans ce cas, le logement pour la réception de l'écran transparent en matériau radio-protecteur est avantageusement délimité par deux parois longitudinales en regard qui sont reliées par deux parois d'extrémité transversales et, du côté de la surface extérieure de l'enveloppe, par une paroi extérieure munie d'une fenêtre autorisant un accès visuel audit écran transparent.

Par ailleurs, la surface extérieure de l'enveloppe est avantageusement munie d'empreintes latérales pour optimiser la prise en main du dispositif, lesquelles empreintes latérales sont diamétralement opposées et sont ménagées de part et d'autre de l'écran de radioprotection transparent.

De plus, la pièce extérieure en matériau plastique élastomère comporte avantageusement, du côté de l'ouverture avant de l'enveloppe tubulaire et à l'opposé de l'écran transparent, une troncature locale en forme générale de biseau, pour faciliter le positionnement de l'extrémité avant d'une seringue rapportée par rapport au point d'injection d'un patient.

L'invention sera encore illustrée, sans être aucunement limitée, par la description suivante d'une forme de réalisation particulière en relation avec les dessins annexés dans lesquels :
- la figure 1 est une vue générale et en perspective du dispositif de radioprotection, rapporté sur une seringue ;
- la figure 2 représente, vus de côté, le dispositif de radioprotection et sa seringue associée ;
- la figure 3 est une vue en coupe du dispositif de radioprotection illustré sur les figures 1 et 2, selon un plan de coupe longitudinal passant par son écran transparent ;
- la figure 4 montre le dispositif de radioprotection vu de dessus ;
- la figure 5 est une vue d'extrémité arrière du dispositif de radioprotection illustré sur la figure 4 ;
- la figure 6 est une vue en coupe transversale du dispositif de radioprotection, selon le plan de coupe VI-VI de la figure 2 ;
- la figure 7 est une vue en perspective de la pièce intérieure tubulaire constitutive du dispositif de radioprotection ;
- la figure 8 est une vue d'extrémité de la pièce intérieure tubulaire selon la figure 7.

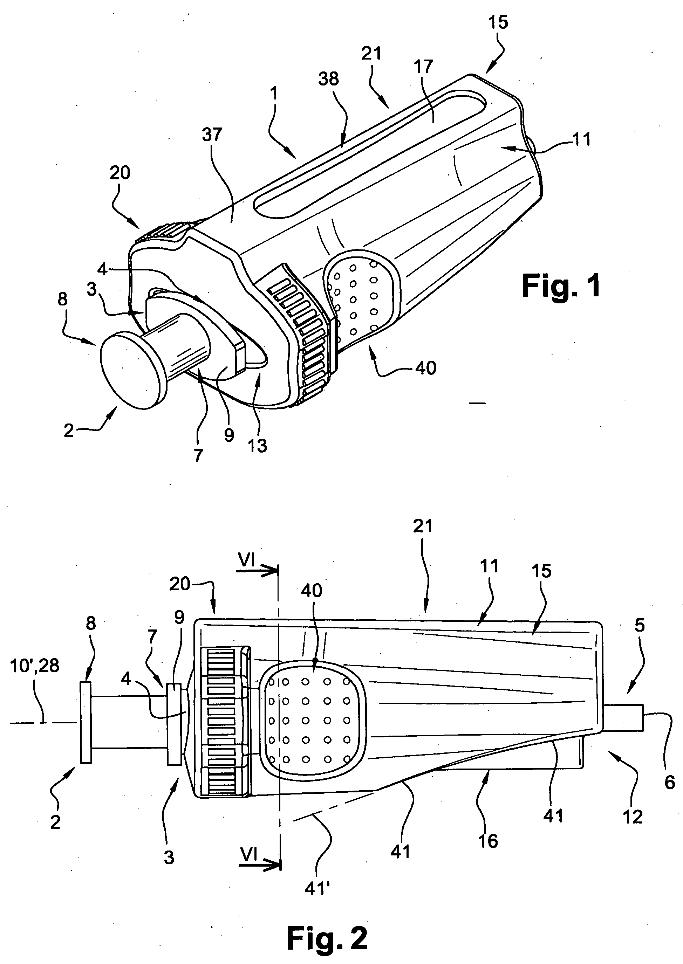

Tel qu'illustré par les figures 1 et 2, le dispositif de radioprotection 1 est destiné à équiper une seringue 2 servant à l'injection de produit(s) radioactif(s).

De manière classique, la seringue 2 comporte un corps cylindrique 3 muni - d'une surface extérieure annulaire 4, - d'une extrémité avant 5, comportant un orifice 6 d'aspiration et d'éjection d'un liquide, et - d'une extrémité arrière 7, au niveau de laquelle est rapporté un piston 8 et qui est munie d'une collerette arrière 9.

Le dispositif de radioprotection 1, dénommé encore «protège-seringue », consiste en une enveloppe tubulaire de radioprotection qui est rapportée par emmanchement autour de la surface extérieure annulaire 4 du corps cylindrique 3 de la seringue 2.
Ce dispositif protège-seringue 1 a pour fonction d'atténuer les rayonnements ionisants émis par le liquide aspiré dans le corps 3 de la seringue 2, et il protège ainsi les opérateurs (et aussi les patients) envers les rayonnements ionisants émis par ce liquide.

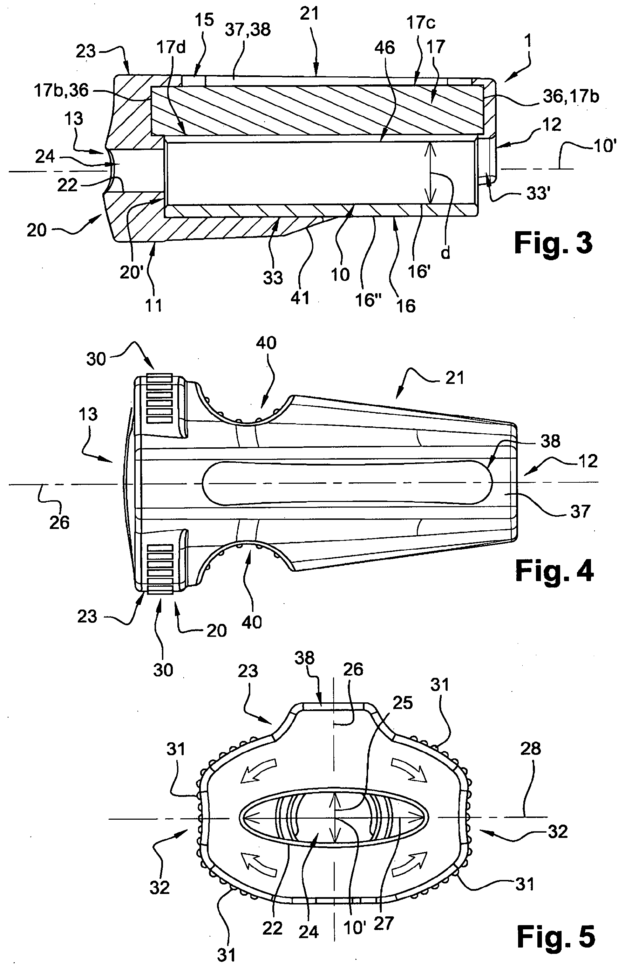

L'enveloppe tubulaire 1 comporte deux surfaces opposées 10 et 11, à savoir :
- une surface intérieure 10, définissant un axe longitudinal 10' et destinée à recouvrir au moins une partie de la longueur de la surface extérieure 4 du corps cylindrique 3 de la seringue 2 (figure 3), et
- une surface extérieure 11, destinée à la prise en main par l'opérateur lors de la manipulation de la seringue 2.

Cette enveloppe tubulaire 1 comporte deux ouvertures 12 et 13, au niveau desquelles débouche la surface intérieure 10, à savoir :
- une ouverture avant 12, au travers de laquelle débouche l'extrémité avant 5 du corps cylindrique 3 de la seringue 2, et
- une ouverture arrière 13, pour l'introduction et l'extraction dudit corps cylindrique 3, ainsi que pour la manoeuvre axiale du piston 8.

Dans le mode de réalisation illustré, l'enveloppe tubulaire 1 est constituée par trois pièces 15, 16 et 17, qui sont assemblées par emboîtement de formes complémentaires, à savoir :
- une pièce extérieure 15 en matériau élastomère, de forme générale tubulaire, constituant notamment la surface extérieure 11 de l'enveloppe tubulaire 1,
- une pièce intérieure 16 en matériau métallique radio-protecteur, également de forme générale tubulaire, logée dans ladite pièce extérieure 15 et comportant une surface intérieure 16' constituant un tronçon circulaire de la surface intérieure 10 de l'enveloppe tubulaire 1, dont le diamètre est désigné par le repère d (figure 3), et
- un écran transparent 17, en matériau radio-protecteur, pour accéder visuellement au corps cylindrique 3 de la seringue.

La pièce extérieure 15 est en forme de gaine qui constitue ici la quasi-intégralité de la surface extérieure 11 de l'enveloppe tubulaire 1.

Cette pièce extérieure 15 est réalisée dans un matériau élastomère (avantageusement du silicone) dont la valeur de dureté Shore A est avantageusement comprise entre 30 et 80, de préférence de l'ordre de 40 à 50, et de préférence encore de l'ordre de 45.

Cette pièce extérieure tubulaire 15 est avantageusement obtenue monobloc par un procédé de moulage par injection.

Sur le plan structurel, la pièce extérieure 15 peut être divisée en deux parties 20 et 21, à savoir :
- une partie annulaire constrictive 20, située du côté de l'ouverture arrière 13 de l'enveloppe tubulaire 1, et
- une partie annulaire avant 21, au niveau de laquelle sont rapportés la pièce intérieure 16 et l'écran transparent 17.

La partie annulaire constrictive 20 comprend deux surfaces 22 et 23 :
- une surface intérieure 22, constituant un tronçon arrière de la surface intérieure 10 de l'enveloppe tubulaire 1 (figures 3 et 5), et
- une surface extérieure 23, constituant un tronçon arrière de la surface extérieure 11 de cette même enveloppe tubulaire 1.

La surface intérieure 22 de la partie annulaire constrictive 20 est déformable élastiquement, pour venir exercer une force de serrage autour du corps cylindrique 3 de la seringue 2 rapportée (figures 1 et 2).

A cet effet, tel qu'illustré sur la figure 5, cette surface intérieure 22 délimite ici un orifice 24 de forme générale allongée, rappelant une forme oblongue ou ovale allongée.

L'orifice 24 correspondant peut être défini par deux dimensions radiales, à l'équerre l'une par rapport à l'autre : l'une 25, de petite dimension, s'étendant dans un premier plan radial de symétrie 26 passant par l'écran de radioprotection 17, et l'autre 27, de grande dimension, passant par un second plan radial de symétrie 28 (orienté perpendiculairement au premier plan 26 précité).

Pour permettre une déformation et une action de serrage optimales, il est avantageusement prévu, d'une part, que la petite dimension 25 est inférieure au diamètre d du tronçon circulaire 16' de l'enveloppe 1 défini par la pièce intérieure 16 et, d'autre part, que la grande dimension 27 est supérieure à ce diamètre d.
A titre indicatif, la petite dimension 25 présente un ratio compris entre 0,9 et 0,5 par rapport au diamètre d, et la grande dimension 27 présente un ratio compris entre 1,5 et 2 par rapport au diamètre d.

Pour faciliter la déformation de cette partie annulaire constrictive 20, sa surface extérieure 23 comporte ici deux zones d'appui 30 qui sont agencées de manière diamétralement opposée et sur le plan de symétrie 28 passant par la grande dimension 27 de l'orifice 24.

Les zones d'appui 30 sont chacune munies d'une pluralité de nervures ou stries 31, juxtaposées et orientées parallèlement à l'axe longitudinal 10', pour optimiser le positionnement des doigts de l'opérateur.
Elles comportent encore chacune une partie centrale concave 32, centrées sur le plan 28 passant par la grande dimension 27 de l'orifice 24, pour encore faciliter la prise en doigts de l'opérateur, et réduire localement l'épaisseur de cette partie annulaire constrictive (figure 5). Cette caractéristique permet en particulier de limiter la force nécessaire à la déformation de cette partie annulaire constrictive 20.

En fonctionnement, la partie annulaire constrictive 20 est ainsi apte à subir une déformation élastique entre deux configurations :
- une configuration active, dans laquelle sa surface intérieure 22 est apte à venir exercer une force de serrage autour du corps cylindrique 3 d'une seringue rapportée 1, pour générer une force de frottement s'opposant à la translation de l'enveloppe tubulaire 1 par rapport audit corps cylindrique 3 (figures 1 et 2), et
- une configuration inactive, obtenue par une déformation de ladite partie annulaire constrictive 20, dans laquelle sa surface intérieure 22 génère une force de serrage réduite autour dudit corps cylindrique 3, pour limiter, voire supprimer, la force de frottement s'opposant à la translation de l'enveloppe tubulaire 1 par rapport au corps cylindrique 3 (non représentée).

Dans la configuration active précitée, la seringue 2 est convenablement maintenue au sein du dispositif de radioprotection 1, par le serrage de la surface intérieure annulaire 22 de la partie constrictive 20 (alors que la surface 16' de la pièce intérieure métallique 16 n'exerce pratiquement aucune action de serrage sur la seringue).

Dans la forme de réalisation illustrée, la configuration inactive est obtenue par un écrasement manuel des deux zones d'appui 30, en direction l'une de l'autre.
Cette action d'écrasement génère une diminution de la valeur de la grande dimension 27 et, simultanément, une augmentation de la valeur de la petite dimension 25. L'orifice 24 prend alors une forme générale circulaire, ce qui réduit, voir supprime, la force de contact entre la surface intérieure 22 de la partie constrictive 20 et le corps cylindrique 3 de la seringue 2. Cette seringue 2 peut alors facilement être retirée de son enveloppe protectrice 1.

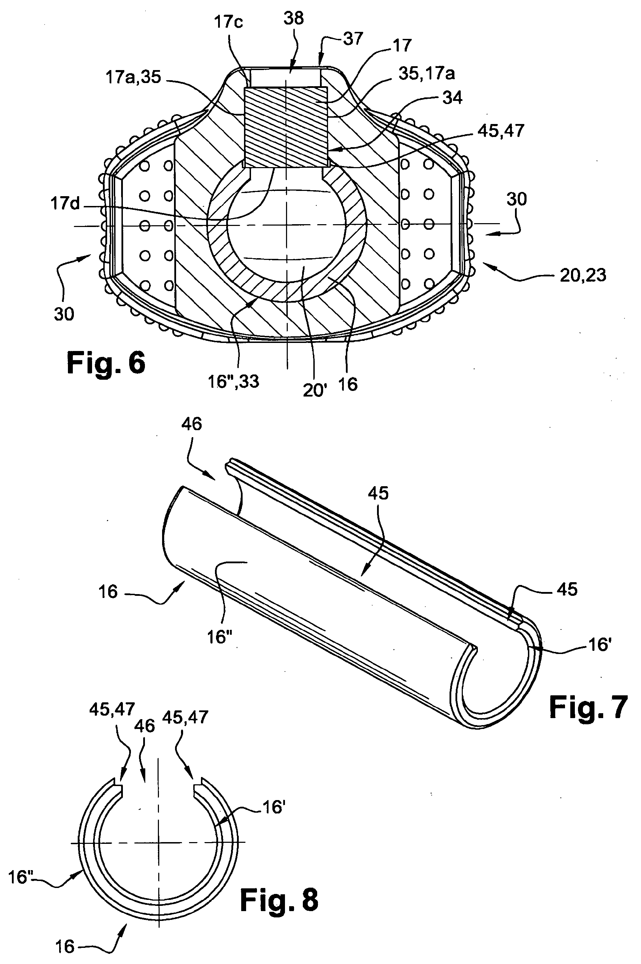

D'autre part, tel qu'illustré sur les figures 3 et 6, la partie avant 21 de la pièce extérieure 15 comporte une surface intérieure pouvant être divisée en deux parties, à savoir :
- une partie inférieure 33 présentant une section en arc de cercle, constituant un logement destiné à recevoir la pièce intérieure tubulaire 16, et
- une partie supérieure 34, formant un logement recevant l'écran transparent de radioprotection 17.

Dans son logement de réception 33, la pièce intérieure 16 est ici verrouillée en translation par deux surfaces de butée (figure 3).
Pour cela, ce logement 33 est muni, du côté de l'ouverture avant 12, d'une nervure saillante 33' formant une surface de butée avant. Et à l'autre extrémité, la partie annulaire constrictive 20 comporte une surface avant 20' définissant une surface de butée arrière.

Le logement de réception 34 pour l'écran transparent 17 est ménagé dans le prolongement du logement inférieur 33 ; il est quant à lui de forme générale parallélépipédique, qui comprend - deux parois longitudinales en regard 35, prolongeant chacune la partie inférieure 33, - deux parois d'extrémité transversales 36, reliant lesdites parois longitudinales 35, et - une paroi extérieure 37 munie d'une fenêtre ou lucarne 38 autorisant un accès visuel à l'écran transparent de radioprotection 17.

Du côté de l'extrémité arrière de sa surface extérieure 11, la partie avant 21 de la pièce extérieure 15 comporte encore deux empreintes latérales concaves 40, pour optimiser la prise en main de l'enveloppe tubulaire 1 et de la seringue 2 associée (figure 4).

Ces empreintes latérales 40 sont diamétralement opposées et elles sont ménagées de part et d'autre de l'écran transparent de radioprotection 17 ; elles présentent chacune une section transversale en arc de cercle, dont l'axe s'étend parallèlement au plan de symétrie 26 passant par l'écran transparent 17 (figure 4).

De plus, du côté de son extrémité avant et à l'opposé de l'écran transparent de radioprotection 17, la partie avant 21 de la pièce extérieure 15 comporte encore une troncature locale 41 en forme générale de biseau, visible sur les figures 2 et 3.
Plus précisément, cette troncature locale 41 s'étend dans un plan 41' définissant un angle de l'ordre de 15° avec le plan de symétrie 28 perpendiculaire au plan de symétrie 26 passant par l'écran transparent 17 ; elle peut être en forme d'arc de cercle de flèche peu importante.
Le plan 41' de cette troncature locale 41 passe, au niveau de l'ouverture avant 12, par une droite correspondant au moins approximativement au diamètre de ladite ouverture 12. Cette troncature 41 se termine, au moins approximativement, au niveau de la moitié de la longueur de cette partie avant 21.

Cette troncature 41 permet de faciliter le positionnement de l'extrémité avant de la seringue rapportée 2, par rapport au point d'injection d'un patient.

Sur les figures 2 et 3, la pièce intérieure 16 s'étend en saillie par rapport au plan 41', de sorte qu'une partie avant de sa surface extérieure 16" constitue une partie de la surface extérieure 11 de l'enveloppe tubulaire 1.

A titre indicatif, l'épaisseur de la pièce annulaire constrictive 20 (correspondant à la distance entre ses surfaces intérieure 22 et extérieure 23) est avantageusement comprise entre 0,3 et 1 cm. L'épaisseur de la partie avant 21 de la pièce extérieure 15 (correspondant à la distance entre ses surfaces intérieure 33 et extérieure 11) est avantageusement comprise entre 0,2 et 0,6 cm.

La pièce intérieure 16 visible notamment sur les figures 3 et 6, est représentée de manière isolée sur les figures 7 et 8.

Cette pièce intérieure tubulaire 16 est réalisée en une ou plusieurs couches de matériau radio-protecteur, par exemple en tungstène, en plomb, en tantale, en composé chargé, ou plus généralement en tout matériau de nature à atténuer les rayonnements ionisants.

Elle présente une section transversale en forme générale d'arc de cercle, comportant deux surfaces opposées, l'une intérieure 16', formant le tronçon circulaire de la surface intérieure 10 de l'enveloppe tubulaire 1, et l'autre extérieure 16", dont le diamètre correspond au diamètre défini par le logement dédié 33 de la pièce extérieure 15.

Ces deux surfaces opposées 16' et 16" se rejoignent au niveau de deux bordures longitudinales 45, en regard et à distance l'une de l'autre, qui définissent une ouverture longitudinale 46 au niveau de laquelle est destinée à être positionné l'écran transparent de radioprotection 17 (figures 3 et 6).

Cette ouverture longitudinale 46 s'étend ici sur toute la longueur de la pièce intérieure 16.

De manière à optimiser le positionnement et le maintien de l'écran transparent de radioprotection 17, les deux bordures longitudinales 45 précitées sont chacune munies d'une feuillure 47, en regard l'une de l'autre, pour le positionnement de la partie intérieure de cet écran transparent 17.
A titre indicatif, l'épaisseur de cette pièce intérieure 16 est constante et avantageusement comprise entre 0,5 et 15 mm, de préférence entre 1 et 3 mm.

L'écran transparent 17 consiste quant à lui en une tige ou barreau parallélépipédique, dont les dimensions correspondent à celles du logement dédié 34 de la pièce extérieure 15.

Cet écran 17 est réalisé en matériau radio-protecteur transparent tel que du verre plombé ou un matériau plastique transparent chargé en éléments radio-atténuateurs.

Cet écran transparent de radioprotection 17 comporte les faces suivantes :
- deux faces longitudinales latérales 17a, épousant les parois latérales 35 du logement dédié 34,
- deux parois transversales 17b, épousant les parois transversales 36 du logement 34,
- une paroi longitudinale supérieure 17c, épousant la paroi supérieure 37 du logement 34, et
- une paroi longitudinale inférieure 17d, épousant les deux bordures longitudinales 45 munies des feuillures 47 de la pièce intérieure 16.

A titre indicatif, l'épaisseur de cet écran 17 (correspondant à la distance entre ses parois longitudinales supérieure 17c et inférieure 17d) est avantageusement comprise entre 5 et 20 mm.

Les angles de l'écran 17 situés entre la paroi inférieure 17d et les parois longitudinales 17a viennent se positionner et se bloquer dans les feuillures 47 de la pièce intérieure 16 ; et cet ensemble vient se loger dans les logements 33 et 34 adaptés de la pièce extérieure 15.

Le positionnement de la pièce intérieure 16 et de l'écran 17 au sein de la pièce extérieure 15 est réalisé grâce à l'élasticité de cette dernière; et cette élasticité réalise l'assemblage des trois pièces 15, 16 et 17, sans nécessiter d'autres moyens de solidarisation.

Le dispositif protège-seringue selon l'invention a l'intérêt de pouvoir être assemblé ou désassemblé de manière simple et rapide, ce qui est utile notamment pour les opérations de maintenance, de réparation ou de nettoyage de ses différentes parties constitutives 15, 16 et 17.

En fonctionnement, lorsqu'un opérateur souhaite protéger une seringue 2, il lui suffit de choisir et de rapporter une enveloppe radio-atténuatrice 1 adaptée aux dimensions de cette dernière.

A cet effet, il est avantageusement prévu de proposer une gamme de dispositifs protège-seringue, dont les dimensions sont adaptées aux différentes seringues à équiper.

Pour simplifier le choix du dispositif protège-seringue 1 adapté à la seringue 2 à protéger, les pièces extérieures 15 de la gamme peuvent comporter des couleurs distinctives, ce qui est rendu possible de manière très simple du fait de leur réalisation en matière plastique.

Pour rapporter le dispositif protège-seringue 2 choisi, l'opérateur maintient une pression sur les zones d'appui 30, en direction l'une de l'autre ; puis il introduit axialement l'extrémité avant 5 de la seringue 2 jusqu'à ce que sa collerette 9 vienne en butée contre la face arrière de la partie annulaire constrictive 20.

L'opérateur peut alors relâcher les zones d'appui 30, de sorte que la partie annulaire constrictive 20 se resserre automatiquement autour du corps 3 de la seringue 2.
Il peut alors prélever et manipuler le ou les produits radioactifs à injecter au patient au moyen de la seringue 2 protégée, en saisissant l'enveloppe tubulaire 1.

Du fait des matériaux utilisés (en particulier le matériau élastomère), la pièce extérieure 15 apporte une qualité de toucher améliorée, avec notamment une force de frottement élevée ce qui réduit les risques de laisser glisser involontairement la seringue 2.

Le volume de liquide prélevé dans la seringue 2 peut être directement visualisé au travers de l'écran transparent 17.

Si l'ensemble seringue/protège-seringue vient à être lâché par accident, la pièce extérieure 15 absorbe efficacement les chocs, et les risques de détérioration (tant de la seringue 2 que de l'écran transparent 17) sont alors significativement réduits.

Ensuite, pour jeter la seringue 2, il suffit d'exercer une nouvelle pression sur les zones d'appui 30 de la partie annulaire constrictive 20 et, tout en maintenant cette pression, d'appliquer une traction axiale sur la seringue 2 (ou de la laisser glisser par gravité) pour la séparer du dispositif protège-seringue.

A titre de variante de réalisation, la pièce extérieure 15 peut être réalisée dans un matériau plastique radio-atténuateur ; dans ce cas la pièce intérieure 16 ne serait alors plus vraiment utile et pourrait être supprimée.

## Revendications

1. Dispositif de radioprotection destiné à équiper une seringue (2) servant à l'injection de produit(s) radioactif(s), ladite seringue (2) comportant un corps cylindrique (3) muni - d'une surface extérieure (4), - d'une extrémité avant (5) comportant un orifice (6) d'aspiration et d'éjection d'un liquide, et - d'une extrémité arrière (7) au niveau de laquelle est rapporté un piston (8), lequel dispositif de radioprotection comprend une enveloppe tubulaire de radioprotection (1) qui comporte - une surface intérieure (10), destinée à recouvrir au moins une partie de ladite surface extérieure (4) du corps cylindrique (3), - une surface extérieure (11), - une ouverture avant (12) au travers de laquelle est destinée à déboucher ladite extrémité avant (5) dudit corps cylindrique (3), - une ouverture arrière (13) pour l'introduction et l'extraction dudit corps cylindrique (3), ainsi que pour la manoeuvre dudit piston (8), ladite enveloppe (1) comportant une pièce extérieure tubulaire (15), constituant une partie au moins de sa surface extérieure (11) qui est réalisée en matériau plastique **caractérisé en ce que** ladite pièce extérieure tubulaire (15) est réalisée en matériau élastomère, et **en ce qu'**elle comporte une partie annulaire constrictive (20) comprenant une surface intérieure (22) qui est déformable élastiquement, laquelle surface intérieure (22) constitue un tronçon de la surface intérieure (10) de l'enveloppe (1) et est destinée à venir exercer une force de serrage autour du corps cylindrique (3) d'une seringue (2) rapportée, ladite partie annulaire constrictive (20) étant apte à subir une déformation élastique entre deux configurations :
- une configuration active, dans laquelle sa surface intérieure (22) est apte à venir exercer une force de serrage autour du corps cylindrique (3) d'une seringue rapportée (2), pour générer une force de frottement s'opposant à la translation de l'enveloppe (1) par rapport audit corps cylindrique (3), et
- une configuration inactive, obtenue par déformation, dans laquelle sa surface intérieure génère une force de serrage réduite autour dudit corps cylindrique (3), pour limiter, voire supprimer, la force de frottement s'opposant à la translation de l'enveloppe (1) par rapport audit corps cylindrique de seringue (3).

2. Dispositif de radioprotection selon la revendication 1, **caractérisé en ce que** le matériau élastomère constituant la pièce extérieure tubulaire (15) comporte une valeur de dureté Shore A comprise entre 30 et 80.

3. Dispositif de radioprotection selon l'une quelconque des revendications 1 ou 2, **caractérisé en ce que** la partie annulaire constrictive (20) constitue l'ouverture arrière (13) de l'enveloppe tubulaire (1).

4. Dispositif de radioprotection selon la revendication 1, **caractérisé en ce que** la surface intérieure (10) de l'enveloppe (1) comporte un tronçon (16') présentant une section circulaire définissant un diamètre (d) déterminé, prolongeant la partie annulaire constrictive (20), et **en ce que**, au repos, la surface intérieure (22) de ladite partie annulaire constrictive (20) définit un orifice (24) de forme générale allongée, comportant d'une part, une petite dimension (25) qui est inférieure audit diamètre (d) dudit tronçon circulaire (16') de l'enveloppe (1) et, d'autre part, une grande dimension (27) qui est supérieure audit diamètre (d).

5. Dispositif de radioprotection selon la revendication 4, **caractérisé en ce que** la partie annulaire constrictive (20) comporte, au niveau de sa surface extérieure (23), deux zones d'appui (30) qui sont agencées de manière diamétralement opposée et sur un plan (28) passant par la grande dimension (27) de l'orifice (24) de ladite partie annulaire constrictive (20), pour faciliter la déformation de cette dernière dans sa configuration inactive.

6. Dispositif de radioprotection selon l'une quelconque des revendications 1 à 5, **caractérisé en ce que** l'enveloppe tubulaire (1) comprend un écran en matériau radio-protecteur transparent (17), pour accéder visuellement à la surface extérieure (4) du corps cylindrique (3) de la seringue (2).

7. Dispositif de radioprotection selon l'une quelconque des revendications 1 à 6, **caractérisé en ce que** l'enveloppe (1) comporte encore une pièce intérieure tubulaire (16) réalisée en matériau radio-protecteur, logée dans la pièce extérieure (15) et destinée à constituer une partie au moins de la surface intérieure (10) de ladite enveloppe (1).

8. Dispositif de radioprotection selon les revendications 6 et 7 prises en combinaison, **caractérisé en ce que** la pièce intérieure tubulaire (16) comporte, sur une partie au moins de sa longueur, une ouverture longitudinale (46) en regard de laquelle est positionné l'écran transparent en matériau radio-protecteur (17).

9. Dispositif de radioprotection selon la revendication 8, **caractérisé en ce que** la pièce intérieure (16) comporte deux bordures longitudinales (45) délimitant l'ouverture longitudinale (46), lesquelles bordures longitudinales (46) sont chacune munies d'une feuillure (47) pour l'emboîtement de la surface intérieure (17d) de l'écran transparent (17).

10. Dispositif de radioprotection selon l'une quelconque des revendications 6 à 9, **caractérisé en ce que** la pièce extérieure (15) comporte un logement (34) pour la réception de l'écran transparent (17), et le cas échéant un logement (33) pour la réception de la pièce intérieure (16), pour leur assemblage sans moyens complémentaires de solidarisation.

11. Dispositif de radioprotection selon la revendication 10, **caractérisé en ce que** le logement (34) pour la réception de l'écran en matériau radio-protecteur transparent (17), est délimité par deux parois longitudinales (35) en regard qui sont reliées par deux parois d'extrémité transversales (36) et, du côté de la surface extérieure (11) de l'enveloppe (1), par une paroi extérieure (37) munie d'une fenêtre (38) autorisant un accès visuel audit écran transparent (17).

## Patentansprüche

1. Strahlenschutzvorrichtung, die dazu bestimmt ist, eine Spritze (2) auszurüsten, die einen zylindrischen Körper (3) aufweist, der - eine äußere Oberfläche (4), - ein vorderes Ende (5) mit einem Ansaug-und Ausstoßloch (6) für eine Flüssigkeit und - einem hinteren Ende (7), in deren Bereich ein Kolben (8) angebracht ist, aufweist, wobei die Schutzvorrichtung eine rohrförmige Strahlenschutzhülle (1) aufweist, die - eine innere Oberfläche (10), die dazu bestimmt ist, wenigstens einen Teil der äußeren Oberfläche (4) des zylindrischen Körpers (3) zu bedecken, - eine äußere Oberfläche (11), - eine vordere Öffnung (12), durch die hindurch das vordere Ende (5) des zylindrischen Körpers (3) münden soll, - eine hintere Öffnung (13) zum Einsetzen und Herausnehmen des zylindrischen Körpers (3) sowie zum Betätigen des Kolbens (8) aufweist, wobei die Hülle (1) ein rohrförmiges äußeres Stück (15) aufweist, das wenigstens einen Teil von deren Oberfläche bildet, die aus einem Plastikmaterial hergestellt ist, **dadurch gekennzeichnet, daß** das rohrförmige äußere Stück (15) aus einem elastomeren Material hergestellt ist und daß es einen ringförmigen einengenden Teil (20) aufweist, der eine innere Oberfläche (22) aufweist, die elastisch verformbar ist, wobei die innere Oberfläche (22) einen Abschnitt der inneren Oberfläche (10) der Hülle (1) bildet und dazu bestimmt ist, eine Klemmkraft um den zylindrischen Körper (3) einer eingesetzten Spritze (2) herum auszuüben, wobei der ringförmige einengende Teil (20) dazu geeignet ist, einer elastischen Verformung zwischen zwei Konfigurationen unterzogen zu werden:
- einer aktiven Konfiguration, in der dessen innere Oberfläche (22) geeignet ist, eine Klemmkraft um den zylindrischen Körper (3) einer eingesetzten Spritze (2) herum auszuüben, um eine Reibungskraft zu erzeugen, die sich einer Verlagerung der Hülle (1) gegenüber dem zylindrischen Körper (3) der Spritze entgegenstellt, und
- einer nicht aktiven Konfiguration, die durch Verformung erhalten wird, in der dessen innere Oberfläche eine geringere Klemmkraft um den zylindrischen Körper (3) einer eingesetzten Spritze (2) herum ausübt, um die Reibungskraft, die sich einer Verlagerung der Hülle (1) gegenüber dem zylindrischen Körper (3) der Spritze entgegenstellt, zu verringern oder sogar zu unterdrücken.

2. Strahlenschutzvorrichtung gemäß Anspruch 1, dadurch gekenntzeichnet, daß das das rohrförmige äußere Stück (15) bildende elastomere Material eine Shore-A-Härte zwischen 30 und 80 aufweist.

3. Strahlenschutzvorrichtung gemäß irgendeinem der Ansprüche 1 oder 2, **dadurch gekennzeichnet, daß** der ringförmige einengende Teil (20) die hintere Öffnung (13) der rohrförmigen Hülle (1) bildet.

4. Strahlenschutzvorrichtung gemäß Anspruch 1, **dadurch gekennzeichnet, daß** die innere Oberfläche (10) der Hülle (1) einen Abschnitt (16') aufweist, der einen kreisförmigen, einen Durchmesser (d) bestimmenden Querschnitt aufweist, der den ringförmigen einengenden Teil (20) verlängert, und daß die innere Oberfläche (22) des rohrförmigen einengenden Teils (20) im Ruhezustand ein Loch (24) mit länglicher Form aufweist, das einerseits eine kleine Abmessung (25), die kleiner als der Durchmesser (d) des kreisförmigen Abschnitts (16') der Hülle (1) ist, und andererseits eine große Abmessung (27), die größer als der Durchmesser (d) ist, aufweist.

5. Strahlenschutzvorrichtung gemäß Anspruch 4, **dadurch gekennzeichnet, daß** der ringförmige einengende Teil (20) im Bereich seiner äußeren Oberfläche (23) zwei Druckzonen (30) aufweist, die diametral gegenüberliegend und in einer durch die große Abmessung (27) des Lochs (24) des besagten ringförmigen einengenden Teils (20) gehenden Ebene angeordnet sind, um die Verformung des letzteren in dessen inaktive Konfiguration zu erleichtern.

6. Strahlenschutzvorrichtung gemäß irgendeinem der Ansprüche 1 bis 5, **dadurch gekennzeichnet, daß** die rohrförmige Hülle (1) eine Abschirmung aus einem durchsichtigen Strahlenschutzmaterial (17) aufweist, um sichtmäßig zur äußeren Oberfläche (4) des zylindrischen Körpers (3) der Spritze (2) zu gelangen.

7. Strahlenschutzvorrichtung gemäß irgendeinem der Ansprüche 1 bis 6, **dadurch gekennzeichnet, daß** die Hülle (1) noch ein inneres rohrförmiges, aus einem Strahlenschutzmaterial hergestelltes Stück (16) aufweist, das im äußeren Stück (15) untergebracht ist und dazu bestimmt ist, wenigstens einen Teil der inneren Oberfläche (10) der Hülle (1) zu bilden.

8. Strahlenschutzvorrichtung gemäß Anspruch 6 und 7 in Kombination, **dadurch gekennzeichnet, daß** das innere rohrförmige Stück (16) auf wenigstens einem Teil seiner Länge eine längliche Öffnung (46) aufweist, vor der die Abschirmung aus einem durchsichtigen Strahlenschutzmaterial (17) positioniert ist.

9. Strahlenschutzvorrichtung gemäß Anspruch 8, **dadurch gekennzeichnet, daß** das innere Stück (16) zwei die längliche Öffnung (46) begrenzende Längsränder (45) aufweist, wobei die beiden Längsränder (46) jeweils mit einem Falzbereich (47) zum Einbringen der inneren Oberfläche (17d) der durchsichtigen Abschirmung (17) versehen sind.

10. Strahlenschutzvorrichtung gemäß irgendeinem der Ansprüche 6 bis 9, **dadurch gekennzeichnet, daß** das äußere Stück (15) eine Aufnahme (34) zum Aufnehmen der durchsichtigen Abschirmung (17) und gegebenenfalls eine Aufnahme (33) zum Aufnehmen des inneren Stücks (16) für deren Zusammenbau ohne komplementäre Befestigungsmittel aufweist.

11. Strahlenschutzvorrichtung gemäß Anspruch 10, **dadurch gekennzeichnet, daß** die Aufnahme (34) zum Aufnehmen der durchsichtigen Abschirmung aus einem durchsichtigen Strahlenschutzmaterial (17) durch zwei gegenüberliegende Längswandungen (35) begrenzt ist, die durch zwei an den Enden liegende Querwandungen (36) und, auf der Seite der äußeren Oberfläche (11) der Hülle (1), durch eine ein einen sichtmäßigen Zugang zur durchsichtigen Abschirmung (17) ermöglichendes Fenster (38) aufweisende äußere Wandung (37) verbunden sind.

## Claims

1. A radiation protection device intended to be fitted onto a syringe (2) for injecting radioactive material(s), said syringe (2) having a cylindrical body (3) provided with - an outer surface (4), - a front end (5), including a liquid suction and ejection orifice (6), and - a rear end (7), at which is inserted a piston (8), said radiation protection device includes a radiation protection tubular housing (1) which has - an inner surface (10), intended to cover at least a portion of said outer surface (4) of the cylindrical body (3), - an outer surface (11), - a front opening (12), through which is intended to open out said front end (5) of said cylindrical body (3), - a rear opening (13), for the introduction and extraction of said cylindrical body (3), and for the operation of said piston (8), said housing (1) including a tubular outer part (15), forming a portion at least of its outer surface (11), which is made of plastic material, **characterized in that** said tubular outer part (15) is made of elastomeric material, and **in that** it includes a constrictive annular portion (20) comprising an inner surface (22) that is resiliently deformable, said inner surface (22) forming a section of the inner surface (10) of the housing (1) and being intended to exert a clamping force around the cylindrical body (3) of an inserted syringe (2), said constrictive annular portion (20) being capable of undergoing a resilient deformation between two configurations:
- an active configuration, in which its inner surface (22) is adapted to exert a clamping force around the cylindrical body (3) of an inserted syringe (2), to generate a friction force opposing to the translation of the housing (1) with respect to said cylindrical body (3), and
- an inactive configuration, obtained by deformation, in which its inner surface generates a reduced clamping force around said cylindrical body (3), to limit, or even cancel, the friction force opposing to the translation of the housing (1) with respect to said syringe cylindrical body (3).

2. The radiation protection device according to claim 1, **characterized in that** the elastomeric material forming the tubular outer part (15) has a Shore A hardness value comprised between 30 and 80.

3. The radiation protection device according to any one of claims 1 or 2, **characterized in that** the constrictive annular portion (20) forms the rear opening (13) of the tubular housing (1).

4. The radiation protection device according to claim 1, **characterized in that** the inner surface (10) of the housing (1) includes a section (16') of circular cross-section defining a determined diameter (d), extending the constrictive annular portion (20), and **in that**, at rest, the inner surface (22) of said constrictive annular portion (20) defines an orifice (24) of generally elongated shape, including, on the one hand, a small dimension (25) that is lower than the diameter (d) of said circular section (16') of the housing (1) and, on the other hand, a great dimension (27) that is higher than said diameter (d).

5. The radiation protection device according to claim 4, **characterized in that** the constrictive annular portion (20) includes, at its outer surface (23), two pressure areas (30) that are arranged in a diametrically opposed manner and on a plane (28) passing through the great dimension (27) of the orifice (24) of said constrictive annular portion (20), to facilitate the deformation of the latter in its inactive configuration.

6. The radiation protection device according to any one of claims 1 to 5, **characterized in that** the tubular housing (1) comprises a screen made of transparent radiation protective material (17), for visual access to the outer surface (4) of the cylindrical body (3) of the syringe (2).

7. The radiation protection device according to any one of claims 1 to 6, **characterized in that** the housing (1) also includes a tubular inner part (16) made of radiation protective material, accommodated in the outer part (15) and intended to form a portion at least of the inner surface (10) of said housing (1).

8. The radiation protection device according to claims 6 and 7, taken in combination, **characterized in that** the tubular inner part (16) includes, over a portion at least of its length, a longitudinal opening (46) opposite which is positioned the transparent screen made of radiation protective material (17).

9. The radiation protection device according to claim 8, **characterized in that** the inner part (16) includes two longitudinal edges (45) delimiting the longitudinal opening (46), said longitudinal edges (46) being each provided with a rebate (47) for the nesting of the inner surface (17d) of the transparent screen (17).

10. The radiation protection device according to any one of claims 6 to 9, **characterized in that** the outer part (15) includes an accommodation (34) for receiving the transparent screen (17), and if need be, an accommodation (33) for receiving the inner part (16), for the assembling thereof without complementary attaching means.

11. The radiation protection device according to claim 10, **characterized in that** the accommodation (34) for receiving the screen made of transparent radiation protective material (17) is delimited by two opposite longitudinal walls (35) which are connected by two transverse end walls (36), and, on the side of the outer surface (11) of the housing (1), by an outer wall (37) provided with a window (38) enabling a visual access to said transparent screen (17).
